# EUROPÄISCHE PATENTSCHRIFT

(11) **EP 4 057 954 B1**
(45) Veröffentlichungstag und Bekanntmachung des Hinweises auf die Patenterteilung: **27.09.2023**
(21) Anmeldenummer: 20803539.4
(22) Anmeldetag: 06.11.2020
(51) Int. Cl.: A61F 5/02, A61H 1/02

(54) **ORTHOPÄDIETECHNISCHE EINRICHTUNG ZUM UNTERSTÜTZEN EINES UNTEREN RÜCKENS EINES BENUTZERS**
ORTHOPAEDIC DEVICE FOR SUPPORTING A USER'S LOWER BACK
DISPOSITIF ORTHOPÉDIQUE POUR SOUTENIR LE BAS DU DOS D'UN UTILISATEUR

(30) Priorität: 11.11.2019 DE 102019130390
(43) Veröffentlichungstag der Anmeldung: 21.09.2022
(73) Patentinhaber: Ottobock SE & Co. KGaA, 37115 Duderstadt (DE)
(72) Erfinder: MIZERA, Oliver, 37083 Göttingen (DE); VOGEL, Carsten, 37115 Duderstadt (DE); TÜTTEMANN, Markus, 45731 Waltrop (DE); PAPP, Emese, 01097 Dresden (DE)
(74) Vertreter: Gramm, Lins & Partner Patent- und Rechtsanwälte PartGmbB
(86) Internationale Anmeldenummer: PCT/EP2020/081296
(87) Internationale Veröffentlichungsnummer: WO 2021/094220

(56) Entgegenhaltungen:
- WO-A1-2018/194447
- DE-A1-102015 211 523
- US-A- 654 173
- US-A- 4 829 989
- US-A1- 2014 024 978
- US-B1- 7 744 552

## Beschreibung

Die Erfindung betrifft eine orthopädietechnische Einrichtung zum Unterstützen eines unteren Rückens eines Benutzers, wobei die orthopädietechnische Einrichtung wenigstens einen mechanischen Energiespeicher, ein Beckenelement, ein Oberkörperelement und ein Oberschenkelement aufweist, wobei der mechanische Energiespeicher aufladbar und entladbar ist, indem das Oberschenkelelement relativ zu dem Oberkörperelement verschwenkt wird, wobei das Oberkörperelement mittels zweier Schienenelemente an dem Beckenelement angeordnet ist, wobei die Schienenelemente jeweils mit einem ersten Ende um wenigstens eine erste Schwenkachse schwenkbar an dem Beckenelement und mit einem dem erstem Ende entgegengesetzten zweiten Ende um wenigstens eine zweite Schwenkachse schwenkbar an dem Oberkörperelement angeordnet sind.

Eine derartige Vorrichtung ist beispielsweise aus der US 654,173 A und der US 4,829,989 bekannt, wobei letztere den Oberbegriff des Anspruchs 1 offenbart. Die US 7,744,552 B1 beschreibt eine andere Art Vorrichtung, die keine Schienenelement aufweist und über eine an einer Stütze angebrachte Rolle eine Kraft auf den vorderen Rumpfbereich des Trägers aufbringt.

Andere Vorrichtungen zum Unterstützen eines unteren Rückens sind aus dem Stand der Technik seit langem bekannt und sollen insbesondere verwendet werden, um den unteren Rücken zu entlasten, wenn beispielsweise schwere Gegenstände angehoben und getragen werden müssen. Bereits aus der US 443,113 ist eine orthopädietechnische Einrichtung bekannt, die über Blattfederelemente verfügt, deren eines Ende im Schulterbereich und deren anderes Ende am Oberschenkel einer Person befestigt wird. Bückt sich der Träger einer solchen orthopädietechnische Einrichtung wird die Blattfeder gebogenen und damit gespannt. Sie übt dann eine Kraft aus, die den Träger der orthopädietechnischen Einrichtung beim Aufrichten unterstützt.

Eine andere orthopädietechnische Einrichtung ist aus der US 2017/0360588 A1 bekannt. Sie verfügt über ein Beinstützelement mit einer Beinschale zum Anlegen an die Vorderseite eines Oberschenkels. Das Gegenlager ist in Form einer Brustplatte ausgebildet, die gegen den Brustkorb des Trägers der Vorrichtung drückt. Werden die beiden Elemente gegeneinander verschwenkt wird eine Federvorrichtung gespannt, sodass eine Rückstellkraft erzeugt wird, die den Träger der orthopädietechnischen Einrichtung beim Aufrichten helfen soll.

Die WO 2014/195373 A1 beschreibt eine weitere Vorrichtung, mit der der Träger der orthopädietechnischen Einrichtung beim Anheben schwerer Gegenstände unterstützt werden soll. Bei dieser orthopädietechnischen Einrichtung wird der ganze Körper inklusive der Arme und Beine des Trägers unterstützt.

Nachteilig ist jedoch, dass die beschriebenen orthopädietechnischen Einrichtungen nur eine Flexion und Extension der Wirbelsäule des Benutzers erlauben. Dieses Vorbeugen(Flexion) und das wieder Aufrichten (Extension) sind jedoch nicht für alle Situationen ausreichende Bewegungsmöglichkeiten, so dass der Träger einer solchen orthopädietechnischen Einrichtung eingeschränkt wird, wodurch die Einsatzmöglichkeit der orthopädietechnischen Einrichtung begrenzt wird und die Akzeptanz der Vorrichtung beim Benutzer sinkt.

Der Erfindung liegt daher die Aufgabe zugrunde, diese Nachteile zu beheben oder zumindest zu mindern.

Die Erfindung löst die gestellte Aufgabe durch eine orthopädietechnische Einrichtung gemäß dem Oberbegriff des Anspruchs 1, die sich dadurch auszeichnet, dass die zweiten Enden der Schienenelemente im angelegten Zustand der Vorrichtung im Bereich der Schulterblätter des Benutzers angeordnet sind

Das Beckenelement ist bevorzugt als Beckengurt oder Hüftgurt ausgebildet und verläuft folglich vollständig um den Rumpf in Höhe des Beckens oder der Hüfte herum. Zwischen den beiden ersten Enden der beiden Schienenelemente, die an dem Beckenelement angeordnet sind, erstreckt sich ein Teil des Beckenelementes, das vorzugsweise bei der Verwendung der Einrichtung nicht oder zumindest nahezu nicht längenveränderlich ausgebildet ist. In einer konstruktiv einfachen und daher bevorzugten Ausgestaltung ist auch der Abstand zwischen dem ersten Ende und dem zweiten Ende des jeweiligen Schienenelementes bei der Verwendung der Einrichtung nicht oder nahezu nicht längenveränderlich ausgebildet. Gleiches gilt für den Abstand zwischen den beiden zweiten Enden der Schienenelemente. Auf diese Weise ergibt sich ein Parallelogramm, das aufgrund der gelenkigen Anordnung der jeweiligen Teile beweglich ausgebildet ist. In einer bevorzugten Ausgestaltung wird erreicht, dass die Bewegungsfreiheit des Oberkörpers des Benutzers und insbesondere der Wirbelsäule des Benutzers nicht oder zumindest nahezu nicht eingeschränkt wird.

Vorzugsweise ist wenigstens eine der genannten Größen einstellbar ausgebildet. Die jeweilige Größe kann so auf eine Körpergröße des Benutzers angepasst werden. Sie wird nach dem Einstellen auf den individuell gewünschten Wert eingestellt und dann derart fixiert, beispielsweise arretiert, dass sie sich bei der Verwendung der Einrichtung nicht oder zumindest nahezu nicht ändert. Vorzugsweise sind mehrere, besonders bevorzugt alle genannten Größen auf diese Weise einstellbar und arretierbar.

Besonders bevorzugt ist das Oberkörperelement derart an dem Beckenelement angeordnet, dass die Lateralflexion der Wirbelsäule und die Rotation der Wirbelsäule um eine in der Sagittalebene liegende Rotationsachse möglich ist. In diesem Fall ist die Bewegungsfreiheit der Wirbelsäule des Benutzers in keiner Weise eingeschränkt, sodass alle Bewegungen, die der Benutzer der orthopädietechnischen Einrichtung mit seiner Wirbelsäule ohne die orthopädietechnische Einrichtung durchführen kann, auch mit der orthopädietechnischen Einrichtung durchführbar sind.

Unter einer in einer Sagittalebene liegenden Rotationsachse wird insbesondere eine vertikale Rotationsachse verstanden, die bei einem aufrecht stehenden Benutzer in der Medianebene, und damit in der mittleren Sagittalebene liegt. Sie könnte auch als Längsachse der Wirbelsäule bezeichnet werden, wobei die Wirbelsäule eines Menschen aufgrund ihrer geometrischen Ausgestaltung keine Längsachse im mathematischen Sinne aufweist. Selbstverständlich sind auch parallel zu dieser Achse verschobene Rotationsachsen in einer Sagittalebene liegend.

Wird die Bewegungsfreiheit der Wirbelsäule des Benutzers durch die orthopädietechnische Einrichtung nicht eingeschränkt, wird darunter insbesondere verstanden, dass eine Flexion und eine Extension möglich sind. Diese Bewegungen werden auch als Ventralflexion und Dorsalextension oder Inklination und Reklination bezeichnet. Eine Flexion ist das Vorneigen des Oberkörpers und damit der Wirbelsäule und des Kopfes, während die Extension die entgegengesetzte Bewegung ist. Zudem werden in diesem Fall auch andere Bewegungen des Oberkörpers und damit der Wirbelsäule, beispielsweise die Lateralflexion und die Rotation durch die orthopädietechnische Einrichtung nicht eingeschränkt.

Vorzugsweise werden auch Bewegungen der Wirbelsäule, insbesondere eine Neigung der Wirbelsäule zur Seite und/oder nach vorn und hinten und/oder eine Verdrehung der Wirkbelsäule um ihre Längsachse, nicht durch die orthopädietechnische Einrichtung behindert, eingeschränkt oder unmöglich gemacht. Vorzugsweise werden alle diese hier beschriebenen Bewegungen durch die orthopädietechnische Einrichtung weder in ihrem maximalen Bewegungsausschlag, noch in einer Bewegungsreihenfolge eingeschränkt.

Wird das Oberschenkelelement relativ zum Oberkörperelement in eine erste Richtung verschwenkt, wird der mechanische Energiespeicher, der beispielsweise ein elastisches Element, beispielsweise eine Zugfeder, sein kann, mit Energie aufgeladen. Diese erste Richtung entspricht beispielsweise dem Anheben des Oberschenkelelementes beispielsweise um eine Treppenstufe zu erklimmen. Bevorzugt ist die Einrichtung beim Treppensteigen jedoch in einem deaktiven Zustand, so dass beim Treppensteigen keine unterstützende Kraft aufgebracht wird. Auch durch das Vorbeugen (Flexion) des Oberkörpers wird das Oberkörperelement relativ zum Oberschenkelelement entsprechend verschwenkt. Die erste Richtung wird folglich dadurch gekennzeichnet, dass ein Winkel zwischen dem Oberschenkelelement und dem Oberkörperelement durch das Verschwenken abnimmt.

Die Energie, mit der der mechanische Energiespeicher aufgeladen wird, kann beispielsweise eine elastische oder potentielle Energie sein. In diesem Zustand übt der mechanische Energiespeicher vorzugsweise eine Kraft auf das Oberschenkelelement und/oder das Oberkörperelement aus, die in die der ersten Richtung entgegengesetzte zweite Richtung wirkt. Wird das Oberschenkelelement relativ zum Oberkörperelement in diese zweite Richtung verschwenkt, wird der mechanische Energiespeicher entladen und die so abgegebene Energie unterstützt die Bewegung des Oberschenkelelementes relativ zum Oberkörperelement. Bei dieser zweiten Richtung handelt es sich folglich beispielsweise um das Absenken des Oberschenkelelementes oder eine Streckung des Beines oder ein Aufrichten (Extension) des Oberkörpers. In all diesen Bewegungen wird das Oberschenkelelement relativ zum Oberkörperelement in die zweite Richtung verschwenkt.

Möchte der Benutzer der orthopädietechnischen Einrichtung beispielsweise einen schweren Gegenstand heben, geht er dafür in die Knie und greift den Gegenstand. Dabei werden beide Oberschenkel und damit auch das jeweilige Oberschenkelelement relativ zum Oberkörper und damit zum Oberkörperelement in die erste Richtung verschwenkt. Der Winkel zwischen dem Oberschenkel und dem Oberkörper nimmt ab. Dadurch wird der mechanische Energiespeicher potentielle Energie geladen. Zum Anheben des Gegenstandes muss der Benutzer der orthopädietechnischen Einrichtung nun die Beine strecken, wobei der Oberschenkel relativ zum Oberkörper in die entgegengesetzte zweite Richtung verschwenkt wird. Die im mechanischen Energiespeicher gespeicherte potentielle Energie wird dabei abgegeben und unterstützt auf diese Weise die entsprechende Bewegung.

Gemäß der Erfindung verlaufen die ersten Schwenkachsen durch die Hüftgelenke des Benutzers, sodass die ersten Enden der Schienenelemente lateral, also außen, angeordnet sind. Die zweiten Enden der Schienenelemente hingegen sind dorsal, also hinten, am Oberkörperelement positioniert. Die Schienenelemente verlaufen bevorzugt derart, dass das erste Ende relativ zum zweiten Ende um 90° gedreht wird. Dabei sind die Schienenelemente vorzugsweise spiegelsymmetrisch zueinander ausgebildet und angeordnet.

Vorzugsweise verlaufen die zweiten Schwenkachsen zumindest im Wesentlichen in Sagittalebene. Dabei verlaufen sie insbesondere bevorzugt von dorsal nach ventral, also von hinten nach vorn. Auf diese Weise ist es möglich, auch ein Neigen des Körpers und der Wirbelsäule zur Seite zu ermöglichen, ohne die Bewegungsfreiheit einzuschränken.

In einer bevorzugten Ausgestaltung verfügen die Schienenelemente über wenigstens zwei Teilschienen, die aneinander um eine dritte Schwenkachse schwenkbar angeordnet sind. Die dritten Schwenkachsen verlaufen dabei vorzugsweise im Wesentlichen in Sagittalebenen. Besonders bevorzugt verlaufen Sie im angelegten Zustand der orthopädietechnischen Einrichtung im Wesentlichen parallel zu den zweiten Schwenkachsen, wenn der Benutzer der orthopädietechnischen Einrichtung aufrecht steht. Die Schwenkgelenke, die eine Bewegung der Teilschienen relativ zueinander ermöglichen, sind bevorzugt näher am ersten Ende als am zweiten Ende der jeweiligen Schienenelemente angeordnet. Besonders bevorzugt befinden sich diese Gelenke seitlich des Körpers des Benutzers, sodass eine gedachte Verlängerung der dritten Schenkachsen am Körper des Benutzers vorbei führt.

Vorzugsweise sind die zweiten Enden der Schienenelemente im angelegten Zustand der orthopädietechnischen Einrichtung im Bereich der unteren Winkel der Schulterblätter des Benutzers angeordnet. In diesem Bereich findet bei einer Beugung der Wirbelsäule nach rechts oder links die stärkste Abweichung von einer geraden Linie statt, so dass die Gelenke, die in diesem Bereich die zweiten Enden mit dem Oberkörperelement verbinden, optimal positioniert sind.

In einer bevorzugten Ausgestaltung ist ein Abstand zwischen Gelenken, mit denen die zweiten Enden der Schienenelemente am Oberkörperelement angeordnet sind, einstellbar. Die Gelenke sind bevorzugt verlagerbar an dem Oberkörperelement angeordnet. Dies wird beispielsweise erreicht, indem das jeweilige Gelenk an einem Schieber angeordnet ist, der entlang einer Führung, beispielsweise einem Langloch oder einer Kulisse, die in oder an dem Oberkörperelement angeordnet ist, verschiebbar ist.

Bevorzugt sind die zweiten Enden der Schienenelemente derart an dem Oberkörperelement angeordnet, dass sie um zwei verschiedene Schwenkachsen schwenkbar sind, von denen eine vorzugsweise in dorsal-ventral-Richtung und eine in medial-lateral-Richtung verläuft. Die erste dieser beiden Schwenkachsen erlaubt es dem Benutzer der orthopädietechnischen Einrichtung, seinen Oberkörper nach rechts und links zu neigen, während die zweite der beiden Schwenkachsen benötigt wird, um den Oberkörper des Benutzers nach vorn oder hinten zu beugen.

In bevorzugten Ausführungsformen sind die zweiten Enden der Schienenelemente mittels Kugelgelenken an dem Oberkörperelement angeordnet. Dadurch die die Bewegungsfreiheit weiter gesteigert und die Akzeptanz der orthopädietechnischen Einrichtung beim Benutzer weiter erhöht.

Das Oberkörperelement verläuft im angelegten Zustand der orthopädietechnischen Einrichtung vorzugsweise vollständig um den Oberkörper des Benutzers herum. Es ist dabei bevorzugt so formstabil ausgebildet, dass sich sein Durchmesser in medial-lateral-Richtung beim Beugen des Oberkörpers nicht oder im Wesentlichen nicht ändert. Soll der wenigstens eine mechanische Energiespeicher mit Energie aufgeladen werden, muss das Oberkörperelement relativ zu dem Oberschenkelelement verschwenkt werden. Gegebenenfalls muss noch eine Aktivierungsvorrichtung betätigt werden, was beispielsweise durch eine Bewegung des Oberkörperelementes relativ zu dem Beckenelement geschehen kann. Wird der Energiespeicher, der beispielsweise ein Federelement aufweist, aufgeladen, muss dazu eine Kraft ausgeübt werden, die durch das Beugen des Oberkörpers hervorgerufen werden kann. Der Oberkörper übt dann in den genannten Ausführungsbeispielen eine Zugkraft auf das Oberkörperelement aus.

Vorzugsweise weist das Oberkörperelement einen Brustabschnitt auf, der im angelegten Zustand der orthopädietechnischen Einrichtung an wenigstens zwei voneinander beabstandeten Stellen auf unterschiedlichen Seiten eines Brustbeines des Benutzers an der Brust des Benutzers anliegt. Über diese Stellen wird die Kraft von dem Oberkörper auf das Oberkörperelement übertragen. Dies ist natürlich auch möglich, wenn das Oberkörperelement nur an einer einzigen Stelle oder an mehr als zwei Stellen mit der Brust des Benutzers in Kontakt kommt.

Zum Aufladen des Energiespeichers mit Energie wird folglich eine Zugkraft auf das Oberkörperelement durch den Oberkörper und damit durch den Benutzer der orthopädietechnischen Einrichtung ausgeübt. Wird der Energiespeicher entladen, wird eine Zugkraft durch das Oberkörperelement auf den Oberkörper ausgeübt, durch die der untere Rücken des Benutzers beispielsweise beim Aufrichten, unterstützt wird. Die Zugkraft wird dabei vorzugsweise über die Schienenelemente auf das Oberkörperelement und von diesen auf den Oberkörper übertragen. Da die Schienenelemente dorsal, also am Rücken, des Benutzers angeordnet sind, wird die Zugkraft auf den dorsalen Anteil des Oberkörperelementes übertragen und von dort auf den frontalen Anteil des Oberkörperelementes gebracht. Über die Stellen, an denen dieser frontale Anteil mit dem Oberkörper in Kontakt kommt, vorzugsweise also rechts und links des Brustbeines des Benutzers, wird diese Zugkraft auf den Oberkörper übertragen. Dabei stellt die ausreichende Formstabilität sicher, dass es nicht zu einer Einschnürung des Oberkörpers des Benutzers kommt, wenn auf den dorsalen Anteil des Oberkörperelementes die Zugkraft ausgeübt wird. Ist die Formstabilität zu klein, wird die eigentlich von frontal nach dorsal durch die seitlich am Oberkörper vorbeiführenden Teile des Oberkörperelementes wie bei einer Schlinge, auf die eine Zugkraft ausgeübt wird, auf den Oberkörper übertragen. Dabei wird ein Teil der Kraft in eine medial wirkende Kraft übertragen, die zu schmerzhaften Effekten führen kann.

Vorzugsweise ist der Teil des Oberkörperelementes, der den Oberkörper umgibt, nicht vollständig formstabil, sondern weist eine geringe Flexibilität und vorzugsweise Elastizität auf. Dadurch ist sichergestellt, dass die orthopädietechnische Einrichtung und das Oberkörperelement für unterschiedliche Personen mit unterschiedlichen Brustumfängen geeignet ist und in der Größe einstellbar ausgebildet sein kann. Dabei ist es gegebenenfalls ausreichend, einzelne starre und unflexible Elemente auf flexible und vorzugsweise elastische Weise miteinander zu verbinden, also beispielsweise Halbschalen oder Schalenelemente zu verwenden, die Teile des Oberkörpers formstabil und starre umgeben. Alternativ dazu kann das Oberkörperelement auch vollständig ohne starre Elemente ausgebildet sein.

In einer bevorzugten Ausgestaltung verfügt die orthopädietechnische Einrichtung über ein erstes und ein zweites Oberschenkelelement und einen ersten und einen zweiten mechanischen Energiespeicher. Dabei ist der erste mechanische Energiespeicher aufladbar und entladbar, indem das erste Oberschenkelelement relativ zu dem Oberkörperelement verschwenkt wird. Der zweite mechanische Energiespeicher ist aufladbar und entladbar, indem das zweite Oberschenkelelement relativ zu dem Oberkörperelement verschwenkt wird. Durch diese Ausgestaltung ist eine Bewegung der Oberschenkel relativ zum Oberkörperelement unabhängig voneinander möglich. Nur auf den Oberschenkel, der relativ zum Oberkörperelement verschwenkt wurde, wird durch den mechanischen Energiespeicher eine Kraft ausgeübt.

Vorteilhafterweise ist jedes Oberschenkelelement mittels einer Gelenkanordnung um eine Gelenkachse schwenkbar an dem Beckenelement angeordnet. Vorzugsweise wird dabei die Gelenkanordnung so positioniert, dass die Gelenkachse durch ein Hüftgelenk des Benutzers verläuft.

Bevorzugt verfügt das Oberschenkelelement über wenigstens ein Anlageelement zum Anlegen an den Oberschenkel und über wenigstens ein Druckkraftübertragungselement, durch das das Anlageelement mit der Gelenkanordnung verbunden ist. In einer bevorzugten Ausführungsform ist das Druckkraftübertragungselement eine Stange oder eine Schiene und ist besonders bevorzugt ergonomisch geformt. Vorzugsweise ist das Anlageelement durch jeweils längstens ein Druckkraftübertragungselement mit jeder Gelenkanordnung verbunden.

Vorteilhafterweise ist jedes der verwendeten Schienenelemente um wenigstens zwei Schwenkachsen schwenkbar an dem Oberkörperelement angeordnet, wobei wenigstens zwei der Schwenkachsen vorzugsweise senkrecht auf einander stehen.

Besonders bevorzugt ist wenigstens eines der Schienenelemente mittels eines Kugelgelenkes an dem Oberkörperelement angeordnet. Vorzugsweise sind alle Schienenelemente mittels jeweils eines Kugelgelenkes an dem Oberkörperelement angeordnet.

Bevorzugt ist wenigstens ein Schienenelement, besonders bevorzugt jedoch jedes Schienenelement, um eine Bewegungsachse schwenkbar an der jeweiligen Gelenkanordnung, die an dem Beckenelement angeordnet ist, angeordnet, wobei Bewegungsachse vorzugsweise senkrecht auf der Gelenkachse der jeweiligen Gelenkanordnung steht.

Die verschiedenen bewegbaren Ausgestaltungen werden erreicht, dass den Bewegungen des Oberkörpers und insbesondere der Wirbelsäule des Benutzers gefolgt werden kann, und unabhängig von der Position des Oberkörperelementes relativ zum Beckenelement und/oder relativ zu dem wenigstens einen Oberschenkelelement die durch den mechanischen Energiespeicher im geladenen Zustand aufgebrachte Kraft wirken kann.

In einer besonders bevorzugten Ausgestaltung ist das wenigstens eine Schienenelement längenveränderbar ausgebildet. Besonders bevorzugt sind alle Schienenelemente längenveränderbar. Dadurch kann die orthopädietechnische Einrichtung für unterschiedlich große Personen verwendet werden. Bevorzugt ist das längenveränderbare Schienenelement in unterschiedlichen Längeneinstellungen fixierbar, sodass die Länge zwar einstellbar, danach jedoch unveränderbar ist.

Vorteilhafterweise weist der mechanische Energiespeicher wenigstens ein Federelement, einen Druckspeicher, ein pneumatisches und/oder hydraulisches System und/oder einen hydraulischen Energiespeicher auf. Auch elastische Elemente in Form von elastischen Seilen, beispielsweise Gummiseilen, sind vorstellbar. Selbstverständlich sind auch andere Elemente, beispielsweise Gasdruckfedern oder Druckfedern, denkbar, für die eine Umlenkung verwendet wird, um aus der durch die Druckfeder bereitgestellten Druckkraft eine Zugkraft zu machen.

Der mechanische Energiespeicher kann an unterschiedlichsten Positionen der Vorrichtung angeordnet sein. Vorteilhafterweise wird eine Position gewählt, an der der für den Energiespeicher benötigte Bauraum vorhanden ist und der Energiespeicher selbst bei Bewegungen des Beins des Benutzers nicht stört. So kann er beispielsweise am Oberschenkel angeordnet sein.

Zum Anordnen des Oberkörperelementes an dem Oberkörper des Benutzers eignet sich besonders ein Schulterelement zum Anlegen an die Schulter, das beispielsweise in Form von Rucksackträgern oder Hosenträgern ausgebildet sein kann. Es erlaubt eine besonders kleine Bauform der orthopädietechnischen Einrichtung.

Das Oberschenkelelement verfügt vorzugsweise über eine Oberschenkelschale, die vorzugsweise an einem Abstandselement angeordnet ist. Dieses Abstandselement ist als Teil des Oberschenkelelementes vorteilhafterweise mit dem Beckenelement verbunden. Die Längen des beispielsweise als Schiene oder Stab ausgebildeten Druckkraftübertragungselementes und des gegebenenfalls auch als Stab oder Schiene ausgebildeten Abstandselementes sind dabei vorzugsweise so gewählt, dass der gesamte Winkelbereich der möglichen Bewegung des Oberschenkels des Trägers abgedeckt wird. Die Oberschenkelschale ist vorzugsweise gelenkig an dem Abstandselement angeordnet, um einen größtmöglichen Tragekomfort zu erreichen.

In einer bevorzugten Ausgestaltung ist der passive Aktuator eingerichtet, die Kraft in Abhängigkeit von einer Position und/oder einer Orientierung des wenigstens einen Beinstützelementes relativ zum Beckenelement und/oder zum Oberkörperelement aufzubringen.

In einer bevorzugten Ausgestaltung ist an dem Oberschenkelelement, bevorzugt an jedem Oberschenkelelement die Oberschenkelschale zum Anlegen an den Oberschenkel des Benutzers angeordnet. Diese ist vorzugsweise gepolstert ausgebildet, um ein möglichst angenehmes Tragegefühl zu vermitteln. Die Oberschenkelschale ist vorzugsweise mittels eines Kugelgelenkes angeordnet. Dadurch wird eine möglichst umfassende Bewegungsfreiheit gegenüber dem Rest der Einrichtung erreicht, die insbesondere bei Bewegungen des Benutzers von Vorteil ist. Mittels des Kugelgelenkes kann die Oberschenkelschale direkt an einem Schienenelement oder Abstandselement des Oberschenkelelements angeordnet sein. Alternativ dazu ist sie an einem Haltebügel positioniert.

Vorzugsweise ist die Oberschenkelschale relativ zu dem Oberschenkelelement um eine Rotationsachse schwenkbar, bevorzugt gegen eine Kraft eines Federelementes schwenkbar, wobei sich die vorzugsweise Rotationsachse in medial-lateral-Richtung erstreckt. Dies wird besonders einfach durch die Positionierung der Oberschenkelschale an dem Haltebügel erreicht, der um die Rotationsachse schwenkbar an einem weiteren Bauteil des Oberschenkelelementes angeordnet ist.

Vorzugsweise weist die orthopädietechnische Einrichtung ein Oberkörperelement, ein Oberschenkelelement und einen ersten passiven Aktuator auf, der eingerichtet ist, eine Kraft auf das Oberschenkelelement und/oder das Oberkörperelement auszuüben, wenn ein Winkel, der zwischen dem Oberschenkelelement und dem Oberkörperelement eingeschlossen ist, in einem ersten vorbestimmten Winkelbereich liegt. Besonders bevorzugt weist die Einrichtung wenigstens einen zweiten passiven Aktuator auf, der eingerichtet ist, eine Kraft auf das Oberschenkelelement und/oder das Oberkörperelement auszuüben, wenn der Winkel in einem vorbestimmten zweiten Winkelbereich liegt, der vom ersten Winkelbereich verschieden ist.

Durch geschickte Wahl des ersten Winkelbereichs und des zweiten Winkelbereichs kann die erfindungsgemäße Vorrichtung beispielsweise für beide oben beschriebenen Bewegungsabläufe verwendet werden. Bückt sich der Träger der orthopädietechnischen Einrichtung beispielsweise nur ein wenig oder arbeitet in gebeugter Haltung, entspricht dies vorzugsweise dem ersten Winkelbereich, sodass der erste passive Aktuator die benötigte Kraft aufbringt. Bückt sich der Träger der orthopädietechnischen Einrichtung jedoch beispielsweise um etwas vom Boden aufzuheben, entspricht der dabei entstehende Winkel zwischen dem Oberschenkelelement und dem Oberkörperelement vorzugsweise dem zweiten Winkelbereich, sodass der zweite passive Aktuator die Kraft aufbringt.

Vorzugsweise überlappen sich der erste Winkelbereich und der zweite Winkelbereich. Mit anderen Worten existieren folglich Winkel zwischen dem Oberschenkelelement und dem Oberkörperelement, in denen beide passiven Aktuatoren eine Kraft aufbringen.

Vorzugsweise weist der erste passive Aktuator und/oder der zweite passive Aktuator wenigstens einen mechanischen Energiespeicher und/oder einen Dämpfer auf. Dies kann beispielsweise ein elastisches Element, beispielsweise ein Federelement, bevorzugt eine Zugfeder sein. Der erste passive Aktuator und/oder der zweite passive Aktuator kann ausgebildet sein, um über den jeweiligen Winkelbereich, in dem der jeweilige Aktuator die Kraft aufbringt, eine konstante Kraft zu übertragen. Dazu kann der jeweilige Aktuator beispielsweise eine Konstantkraftfeder aufweisen. Alternativ oder zusätzlich dazu kann jedoch der Aktuator auch so ausgebildet sein, dass innerhalb des jeweiligen Winkelbereichs nicht eine konstante Kraft, sondern beispielsweise eine mit sinkendem Winkel steigende Kraft aufgebracht wird. Ein kleiner werdender Winkel bedeutet eine stärkere Beugung, sodass in diesem Fall die vom jeweiligen Aktuator aufgebrachte Kraft steigt, je tiefer sich der Benutzer der Vorrichtung drückt. In einer weiteren Ausgestaltung kann die Kraft auch bei einem Winkel innerhalb des jeweiligen Winkelbereichs ihr Maximum aufweisen und bei größerem Winkel und bei kleineren Winkel abfallen.

Vorzugsweise sind der erste passive Aktuator und der zweite passive Aktuator verschieden ausgebildet. Insbesondere können die elastischen Elemente der beiden Aktuatoren unterschiedliche Elastizitäten, insbesondere verschiedene Federkonstanten, und/oder unterschiedliche Dämpfungen aufweisen. Zudem können sie unterschiedliche Längen aufweisen, wobei die Länge des elastischen Elementes dabei im entspannten Zustand gemessen wird.

Vorzugsweise sind der erste passive Aktuator und/oder der zweite passive Aktuator an wenigstens einem Angriffspunkt am Oberschenkelelement und/oder am Oberkörperelement angeordnet, der jeweils verstellbar ist. Auf diese Weise lässt sich der jeweilige vorbestimmte Winkelbereich, innerhalb dessen der jeweilige Aktuator die Kraft aufbringt, einstellen. Zudem kann eine Vorspannung des jeweiligen passiven Aktuators erreicht werden, sodass auch die Größe der jeweils aufzubringenden Kraft einstellbar ist.

Um unterschiedliche Kräfte aufbringen zu können, ist es dabei von Vorteil, wenn der erste passive Aktuator und der zweite passive Aktuator an unterschiedlichen Angriffspunkten am Oberschenkelelement und/oder am Oberkörperelement angeordnet sind und/oder unterschiedliche Längen aufweisen. Auf diese Weise lässt sich insbesondere bei montierter Vorrichtung leicht erkennen, welcher Aktuator in welchem Winkelbereich seine Kraft aufbringt und/oder welcher Aktuator eine größere oder kleinere Kraft aufbringt. Selbstverständlich ist es auch möglich, die beiden Aktuatoren am gleichen Angriffspunkt angreifen zu lassen oder zwei Aktuatoren mit gleicher Länge zu verwenden. Dies ist beispielsweise dann möglich, wenn die beiden Aktuatoren unterschiedliche Federkonstanten und/oder Elastizitäten aufweisen.

In einer bevorzugten Ausgestaltung verfügt das Oberkörperelement über ein erstes Kraftübertragungselement und das Oberschenkelelement über ein zweites Kraftübertragungselement. Die beiden Kraftübertragungselemente sind in Eingriff und außer Eingriff bringbar. Der erste mechanische Energiespeicher und der zweite mechanische Energiespeicher sind aufladbar und entladbar, in dem das Oberschenkelelement relativ zu dem Oberkörperelement verschwenkt wird, sofern sich das erste Kraftübertragungselement in Eingriff mit dem zweiten Kraftübertragungselement befindet. Andernfalls können das Oberschenkelelement und das Oberkörperelement gegeneinander verschwenkt werden, ohne dass einer der beiden mechanischen Energiespeicher mit Energie aufgeladen werden wird. Durch diese Ausgestaltung ist es möglich, das Oberschenkelelement relativ zum Oberkörperelement zu verschwenken, ohne dass der jeweilige Energiespeicher mit Energie aufgeladen wird. In diesem Zustand wird dann auch keine Kraft vom Energiespeicher, also dem jeweiligen passiven Aktuator, aufgebracht. Dies ist für bestimmte Bewegungsabläufe von Vorteil. Steigt der Benutzer der Vorrichtung beispielsweise eine Treppe, muss er dazu die Oberschenkel und damit auch die an den Oberschenkeln angeordneten Oberschenkelelemente anheben. Mit anderen Worten muss er jeweils ein Oberschenkelelement relativ zum Oberkörperelement verschwenken. Während die beiden Kraftübertragungselemente in diesem Zustand in Eingriff miteinander, würde beim Anheben des Oberschenkelelementes der mechanische Energiespeicher aufgeladen und beim Strecken des Beines auf der nächsthöheren Stufe der Treppe wieder entladen. Soll die Vorrichtung jedoch beim Treppensteigen keine Unterstützung liefern, ist es sinnvoll, bei dieser Bewegung die beiden Kraftübertragungselemente außer Eingriff zugelassen.

In vielen Fällen soll die durch die orthopädietechnische Einrichtung gewährte Unterstützung nur beim Heben oder sich aus der Hocke Aufrichten vorhanden sein. Um dies zu gewährleisten muss sichergestellt werden, dass die beiden Kraftübertragungselemente nur in diesen Zuständen in Eingriff miteinander kommen. Dies lässt sich beispielsweise erreichen, indem ein Beckenelement vorhanden ist, und die beiden Kraftübertragungselemente in Eingriff miteinander gebracht werden, sobald ein Winkel zwischen dem Beckenelement oder einem Bauteil des Beckenelementes und dem Oberkörperelement einen vorbestimmten Grenzwinkel überschreitet.

Vorzugsweise verfügt die Einrichtung daher über ein Beckenelement, wobei das Oberkörperelement relativ zu dem Beckenelement bewegbar angeordnet ist. Das erste Kraftübertragungselement und das zweite Kraftübertragungselement werden in diesem Fall in Eingriff und außer Eingriff gebracht, indem das Oberkörperelement relativ zu dem Beckenelement bewegt wird. Unterschreitet der Winkel zwischen dem Beckenelement und dem Oberkörperelement einen vorbestimmten Grenzwinkel, werden die beiden Kraftübertragungselemente in Eingriff miteinander gebracht. Überschreitet der Winkel danach den vorbestimmten Grenzwinkel, werden die Kraftübertragungselemente wieder außer Eingriff gebracht.

In bevorzugten Ausgestaltungen sind der erste passive Aktuator und der zweite passive Aktuator an jeweils einem Krafteinleitungspunkt an jeweils einem Krafteinleitungshebel angeordnet. Dieser ist vorzugsweise am Beckenelement oder am Oberkörperelement positioniert. Die beiden passiven Aktuatoren greifen in diesen Ausgestaltungen vorzugsweise am Oberschenkelelement an, sind also mit einem ihrer Enden am Oberschenkelelement und mit dem anderen am jeweiligen Krafteinleitungshebel angeordnet. Befinden sich die beiden Kraftübertragungselemente außer Eingriff, können die Krafteinleitungshebel relativ zum Beckenelement frei verschwenkt werden. Wird in diesem Zustand das Oberschenkelelement relativ zum Beckenelement und damit auch relativ zum Oberkörperelement verschwenkt, folgen die Krafteinleitungshebel dieser Verschwenkung, sodass die passiven Aktuatoren und die in ihnen vorzugsweise enthaltenen mechanischen Energiespeicher nicht mit Energie aufgeladen werden. Es entsteht somit keine Kraft und keine Unterstützung.

Greifen die beiden Kraftübertragungselemente jedoch ineinander ein, so sind die Krafteinleitungshebel drehfest am Beckenelement positioniert und können der Schwenkbewegung des Oberschenkelelementes nicht mehr folgen. Der Abstand zwischen dem Krafteinleitungspunkt am Krafteinleitungshebel einerseits und dem Angriffspunkt am Oberschenkelelement andererseits nimmt bei der Bewegung folglich zu, sodass der mechanische Energiespeicher mit mechanischer Energie aufgeladen wird und eine unterstützende Kraft ausübt.

Vorzugsweise ist eine Orientierung und/oder eine Position der beiden Krafteinleitungshebel zueinander und/oder wenigstens einer der beiden, vorzugsweise jedoch beide Krafteinleitungspunkt, verstellbar. Durch die Bewegung, beispielsweise Verschwenkung, der beiden Krafteinleitungshebel relativ zueinander lässt sich der Winkelbereich, in dem der jeweilige passive Aktuator seine Kraft aufbringt, einstellen. Durch eine Verschiebung des Krafteinleitungspunktes am Krafteinleitungshebel, beispielsweise in Richtung auf die Schwenkachse des Oberschenkelelementes relativ zum Beckenelement oder von dieser weg, lässt sich die Stärke der aufzubringenden Kraft einstellen. Durch andere Verstellung des Krafteinleitungspunktes am Krafteinleitungshebel, beispielsweise in Umfangsrichtung bezüglich der oben genannten Schwenkachse, lässt sich auch eine Vorspannung des jeweiligen passiven Aktuators erreichen.

Vorzugsweise ist eine Vorspannung des ersten Aktuators und/oder eine Vorspannung des zweiten Aktuators einstellbar.

Vorzugsweise verläuft die vom ersten Aktuator ausgeübte Kraft und/oder die vom zweiten Aktuator ausgeübte Kraft von dem Winkel abhängt, insbesondere kurvenförmig, besonders bevorzugt sinusförmig.

Bevorzugt weisen die vom ersten Aktuator ausgeübte Kraft und die vom zweiten Aktuator ausgeübte Kraft bei verschiedenen Winkeln ein Maximum auf.

Vorzugsweise ist die von dem jeweiligen Aktuator bei Winkeln kleiner als der jeweilige vorbestimmte Winkelbereich Null, oder im Wesentlichen Null. Der Winkel ist dabei umso kleiner, je stärker der Oberkörper relativ zum Oberschenkel gebeugt wird.

Vorzugsweise greift der erste passive Aktuator und der zweite passive Aktuator an jeweils einem Krafteinleitungshebel an. Die beiden Krafteinleitungshebel sind dabei vorzugsweise längenverstellbar ausgebildet, sodass sich die Größe des vom jeweiligen Aktuators aufgebrachten Drehmoments oder die Stärke der jeweiligen Kraft einstellen lässt. Zudem oder alternativ dazu sind die Krafteinleitungshebel relativ zueinander und/oder relativ zu einem Beckenelement vertretbar ausgebildet, sodass sich die Lage des vorbestimmten ersten Winkelbereiches und/oder die Lage des vorbestimmten zweiten Winkelbereiches einstellen lassen. Beim Beugen des Oberkörperelementes relativ zum Oberschenkelelement wird der jeweilige Aktuator, der beispielsweise ein Federelement sein kann, mit mechanischer Energie aufgeladen und kann so die Kraft aufbringen. Dabei wird der Abstand zwischen dem ersten Ende des jeweiligen Aktuators und dem zweiten Ende des Aktuators größer.

Vorzugsweise verfügt die Einrichtung über einen Anschlag, der beispielsweise an einem Beckenelement angeordnet sein kann, und an den der erste passive Aktuator und/oder der zweite passive Aktuator anschlägt, wenn der jeweilige Krafteinleitungshebel eine bestimmte Position insbesondere relativ zum Oberschenkelelement erreicht hat. Damit wird erreicht, dass der jeweilige Aktuator zwar noch immer gespannt und mit mechanischer Energie aufgeladen ist, diese jedoch vorzugsweise direkt auf die Rotationsachse zwischen dem Oberkörperelement und dem Oberschenkelelement wirkt, sofern der Anschlag auf dieser Rotationsachse angeordnet ist. Damit wird zwar eine Kraft ausgeübt, die jedoch nicht mehr zu einem Drehmoment und damit nicht mehr zu einer Unterstützung des Rückens führt.

Die orthopädietechnische Einrichtung weist bevorzugt ein Gelenk mit einem ersten Gelenkelement, das einen ersten Gelenkarm mit einem ersten Formschlusselement auf, und einem relativ zu dem ersten Gelenkelement verschwenkbaren zweiten Gelenkelement, das einen zweiten Gelenkarm und einen Kraftangriffshebel mit einem zweiten Formschlusselement und einen mechanischen Energiespeicher aufweist, der zwischen dem Kraftangriffshebel und dem zweiten Gelenkarm angeordnet ist, wobei der mechanische Energiespeicher aufladbar und entladbar ist, indem der erste Gelenkarm relativ zu dem zweiten Gelenkarm verschwenkt wird, wenn das erste Formschlusselement mit dem zweiten Formschlusselement in Eingriff ist. Vorzugsweise weist die Einrichtung eine Sicherungseinrichtung auf, durch die sichergestellt ist, dass unabhängig von der Position des ersten Formschlusselementes und des zweiten Formschlusselementes relativ zueinander die beiden Formschlusselemente derart in Eingriff bringbar sind, dass eine von dem aufgeladenen mechanischen Energiespeicher wirkende Kraft von dem zweiten Formschlusselement auf das erste Formschlusselement übertragen wird. Dadurch wird sichergestellt, dass das unkontrollierte Abgeben der Energie beim Abrutschen der Formschlusselemente aneinander, verhindert wird.

Vorzugsweise handelt es sich bei den Formschlusselementen um die bereits beschriebenen Kraftübertragungselemente. Das erste Gelenkelement und das zweite Gelenkelement sind vorzugsweise dem Oberkörperelement und dem Oberschenkelelement oder umgekehrt zugeordnet.

Die Formschlusselemente verfügen über Vertiefungen und/oder Vorsprünge, die so ausgebildet sind, dass die beiden Formschlusselemente formschlüssig miteinander in Eingriff bringbar sind. Bevorzugt handelt es sich um Zahnräder, besonders bevorzugt um Stirnzahnräder.

Prinzipiell sind unterschiedliche Mechanismen denkbar, mit denen die Sicherungseinrichtung die gestellte Aufgabe löst. Vorzugsweise ist die Sicherungseinrichtung eingerichtet, beim in Eingriff Bringen oder nachdem in Eingriff Bringen der beiden Formschlusselemente die Formschlusselemente relativ zueinander zu drehen. Sollten sich die Formschlusselemente zu dem Zeitpunkt, zudem sie miteinander in Eingriff gebracht werden sollen, so zueinander positioniert haben, dass die Vorsprünge und/oder Vertiefungen der beiden Formschlusselemente nicht vollständig, sondern nur in einem kleinen Bereich miteinander in Eingriff gebracht werden können, kann durch die Drehung der beiden Formschlusselemente relativ zueinander diese Relativposition verändert und so ein vollständiger oder zumindest größere Eingriff sichergestellt werden.

Vorzugsweise weisen das erste Formschlusselement und das zweite Formschlusselement stirnseitige Vorsprünge und/oder Vertiefungen auf. Dies ist beispielsweise bei Stirnzahnrädern gegeben. Unter einem Stirnzahnrad wird dabei ein Zahnrad verstanden, dessen Zähne in axialer Richtung hervorstehenden. Bei einem herkömmlichen Zahnrad sind die Zähne am äußeren Umfang des Zahnrades angeordnet und tragen in radialer Richtung hervor. Ein Zahnrad verfügt über eine Rotationsachse, um die es drehbar gelagert ist und bezüglich derer die Begriffe axial und radial zu verstehen sind. Bei einem Stirnzahnrad hingegen befinden sich die Zähne an einer Stirnfläche des Zahnrades und ragen somit in axialer Richtung hervor. Werden zwei derartige Stirnzahnräder miteinander in Eingriff gebracht, greifen vorzugsweise alle Zähne des einen Zahnrades in die Zähne des anderen Zahnrades, sodass der Kontaktbereich deutlich größer ist als dies bei herkömmlichen Zahnrädern, deren Zähne am äußeren Umfang angeordnet sind, ist. Dadurch kann eine größere Kraft übertragen werden.

Werden zwei derartige Stirnzahnräder in Eingriff gebracht, deren Zähne nicht optimal zueinander positioniert sind, kann dies folglich korrigiert werden, indem die beiden Zahnräder relativ zueinander gedreht werden. Die benötigte Drehung ist dabei vorzugsweise klein, insbesondere kleiner als 5°, bevorzugt kleiner als 3°, besonders bevorzugt kleiner als 2°. Dies ist auch bei Formschlusselementen der Fall, die keine Zahnräder mit Zähnen sind, sondern andere Vertiefungen und/oder Vorsprünge aufweisen.

Besonders bevorzugt weist die Sicherungseinrichtung eine aus einem der Formschlusselemente axial hervorstehende Führungsspindel auf, die stirnseitige Vertiefungen und/oder Vorsprünge, insbesondere eine Stirnverzahnung aufweist, die eingerichtet ist, mit dem jeweils anderen Formschlusselement zusammenzuwirken.

Vorteilhafterweise ist die Führungsspindel in axialer Richtung relativ zu dem Formschlusselement, aus dem sie axial hervorstehende, verschiebbar, wobei die Führungsspindel derart ausgebildet ist, das beim axialen Verschieben die Führungsspindel um ihre Längsachse gedreht wird, sodass ein Drehmoment auf das Formschlusselement ausgeübt wird, das mit den stirnseitigen Vorsprüngen und/oder Vertiefungen der Führungsspindel zusammenwirkt. Bei einer derartigen Ausgestaltung steht die Führungsspindel axial aus der Stirnfläche eines der beiden Formschlusselemente hervor, wenn die beiden Formschlusselemente nicht in Eingriff miteinander sind. Sollen die beiden Formschlusselemente nun in Eingriff gebracht werden, wird eines der beiden Formschlusselemente, vorzugsweise das, aus dem keine Führungsspindel hervor steht, in Richtung auf das jeweils andere Formschlusselement bewegt. Dabei kommen zunächst die stirnseitigen Vorsprünge und/oder Vertiefungen, insbesondere die Stirnverzahnung der Führungsspindel mit den stirnseitigen Vertiefungen und/oder Vorsprüngen des anderen Formschlusselementes in Kontakt. Dadurch wird jedoch die Bewegung des anderen Formschlusselementes auf das mit der Führungsspindel versehene Formschlusselement nicht gestoppt, sondern die Führungsspindel wird in axialer Richtung verschoben und in das Formschlusselement, an dem sie angeordnet ist, hineinbewegt.

Vorzugsweise bilden die stirnseitigen Vorsprünge und/oder Vertiefungen, insbesondere die Stirnverzahnung der Führungsspindel mit den stirnseitigen Vertiefungen und/oder Vorsprüngen, insbesondere der Stirnverzahnung ihres Formschlusselement eine durchgehende Verzahnung, wenn die Führungsspindel soweit in ihr Zahnrad verschoben ist, dass sich eine einzige durchgehende Stirnfläche bildet.

Greifen die Vorsprünge und/oder Vertiefungen des anderen Formschlusselementes, das auf die Führungsspindel zu verschoben wurde, nicht optimal in deren Vorsprünge und/oder Vertiefungen ein, über die Führungsspindel ein Drehmoment auf dieses Formschlusselement aus, sodass es in die optimale Position gedreht wird, um in die Vertiefungen und/oder Vorsprünge des anderen Formschlusselementes einzugreifen. Dabei wird diese optimale Position vorzugsweise dann erreicht, wenn die Führungsspindel vollständig in ihrem Formschlusselement versenkt ist. Auf diese Weise wird sichergestellt, dass die Vorsprünge und/oder Vertiefungen der beiden Formschlusselemente in optimaler Position zueinander ineinander eingreifen, unabhängig davon, in welcher Position die beiden Formschlusselemente relativ stehen, wenn sie miteinander in Eingriff gebracht werden sollen.

Alternativ oder zusätzlich dazu weist das erste Formschlusselement und/oder das zweite Formschlusselement wenigstens zwei Teilformschlusselement, beispielsweise wenigstens zwei Teilzahnräder auf, die in axialer Richtung unabhängig voneinander bewegbar sind. Werden die beiden Formschlusselemente in dieser Ausgestaltung aufeinander zu bewegt, um sie miteinander in Eingriff zu bringen, greift eines der Teilformschlusselementes einem Formschlusselement in die Vorsprünge und/oder Vertiefungen des anderen Formschlusselement ein, bevor die anderen Teilformschlusselemente dies tun. Die Teilformschlusselemente sind vorzugsweise in Umfangsrichtung versetzt zueinander angeordnet, sodass die Vorsprünge und/oder Vertiefungen, insbesondere Zähne jedes einzelnen der Teilformschlusselemente untereinander zwar äquidistant angeordnet sind, zwischen den Vorsprüngen und/oder Vertiefungen, insbesondere Zähnen benachbarter Teilformschlusselemente jedoch ein Winkelversatz vorhanden ist. Auf diese Weise ist sichergestellt, dass die Vorsprünge und/oder Vertiefungen unterschiedlicher Teilformschlusselemente unterschiedlich gut in die Vorsprünge und/oder Vertiefungen des jeweils anderen Formschlusselementes eingreifen, wenn die beiden Formschlusselemente miteinander in Eingriff gebracht werden.

Greifen die Vorsprünge und/oder Vertiefungen des ersten Teilformschlusselementes folglich optimal in die Vorsprünge und/oder Vertiefungen des anderen Formschlusselementes ein, ist es ausreichend, um die aufzubringenden Kräfte zu übertragen. Ist dies jedoch nicht der Fall, da die Vorsprünge und/oder Vertiefungen des ersten Teilformschlusselementes beispielsweise nur im Bereich der Spitzen mit den Vertiefungen und/oder Vorsprüngen des anderen Formschlusselementes in Eingriff stehen, greifen die Vorsprünge und/oder Vertiefungen eines der weiteren Teilformschlusselementes besser in das andere Formschlusselement ein. Ist der Kontakt zwischen den Spitzen der Vorsprünge und/oder Vertiefungen des ersten Teilformschlusselementes mit den Spitzen der Vorsprünge und/oder Vertiefungen des anderen Formschlusselementes nicht ausreichend, um die auftretenden Kräfte sicher zu übertragen, kommt es zu einem "Durchrutschen" der beiden Formschlusselemente. Dies wird jedoch bereits nach einer Relativbewegung von wenigen Grad durch die Vorsprünge und/oder Vertiefungen eines der weiteren Teilformschlusselemente abgefangen, die aufgrund des Winkelversatzes zwischen den Vorsprüngen und/oder Vertiefungen unterschiedlicher Teilformschlusselemente besser in die Vorsprünge und/oder Vertiefungen des anderen Formschlusselementes eingreifen.

Vorzugsweise weisen daher die wenigstens zwei Teilformschlusselemente die gleichen Vorsprünge und/oder Vertiefungen, insbesondere die gleiche Zählung auf, die jedoch in Umfangsrichtung gegeneinander versetzt sind. Vorzugsweise ist der Versatz kleiner als 10°, bevorzugt kleiner als 7°, besonders bevorzugt kleiner als 5°. Die gleiche Zähnung bedeutet in diesem Zusammenhang, dass die Zähne die gleiche Tiefe, den gleichen Flankenverlauf, und den gleichen Winkelversatz zueinander aufweisen.

In einer bevorzugten Ausgestaltung sind die wenigstens zwei Teilformschlusselemente in axialer Richtung voneinander beabstandet, wenn das erste Formschlusselement und das zweite Formschlusselement nicht miteinander in Eingriff stehen. Auf diese Weise ist sichergestellt, welches der wenigstens zwei Teilformschlusselemente das erste Teilformschlusselement ist, das mit anderen Formschlusselementen in Kontakt kommen.

Ein Teilformschlusselement ist dabei wie ein Tortenstück geformt. Es verfügt bevorzugt über zwei gerade Kanten und eine kreisbogenförmige Kante. Es handelt sich bevorzugt um ein Kreissegment. An der Stirnfläche befindet sich vorzugsweise die ebenfalls kreissegmentförmige Zähnung.

Vorzugsweise ist das erste Gelenkelement dem Oberkörperelement und das zweite Gelenkelement dem Oberschenkelelement zugeordnet. Die Einrichtung verfügt zudem über ein Beckenelement, wobei die beiden Formschlusselemente in Eingriff und außer Eingriff bringbar sind, in dem das Oberkörperelement relativ zu dem Beckenelement bewegt wird. Dieser Ausgestaltung der Erfindung liegt die Erkenntnis zugrunde, dass der untere Rücken nicht immer dann einer Unterstützung bedarf, wenn ein Winkel zwischen einem Oberkörperelement, das beispielsweise im Brustbereich oder im Rückenbereich des Oberkörpers des Trägers angeordnet wird, und dem Oberschenkel des Trägers einen vorbestimmten Winkel unterschreitet, die beiden Körperteile also gegeneinander verschwenkt werden. Vielmehr ist eine Unterstützung nur dann notwendig, wenn es zu einer Verschwenkung zwischen dem Oberkörper, also beispielsweise den Brustkorb, des Trägers und dem Becken des Trägers kommt. Durch die Ausgestaltung der Vorrichtung in dieser Weise wird erreicht, dass immer dann, wenn diese Verschwenkung zwischen dem Oberkörper und dem Becken des Trägers eintritt, eine unterstützende Kraft ausgeübt wird. Wird hingegen der Oberkörper relativ zum Oberschenkel verschwenkt, ohne dass es zu einer Bewegung des Oberkörpers relativ zum Becken kommt, soll keine Kraft ausgeübt werden. In diesem Fall werden die beiden Zahnräder nicht in Eingriff miteinander gebracht.

Bevorzugt sind an dem Beckenelement oder an dem Oberschenkelelement wenigstens zwei Magnete und an dem jeweils anderen Element wenigstens ein Magnet derart angeordnet, dass sie eine Kraft aufeinander ausüben, deren Richtung wechselt, wenn der Winkel beim Bewegen des Oberkörperelementes relativ zu dem Beckenelement den vorbestimmten Grenzwinkel passiert. In dieser Ausgestaltung verfügt die Verschiebeeinrichtung folglich über die genannten Magnete. An dem Element am Oberkörperelement oder am Beckenelement, an dem wenigstens zwei Magnete angeordnet sind, sind diese bevorzugt in unterschiedlicher Orientierung angeordnet. Dies bedeutet, dass bei wenigstens einem der Magnete der Nordpol und bei wenigstens einem anderen der Südpol in Richtung auf das jeweils andere Element der orthopädietechnischen Einrichtung gerichtet ist.

Ist der Winkel zwischen dem Oberkörperelement und dem Beckenelement größer als der vorbestimmte Grenzwinkel sind die beiden Formschlusselemente nicht miteinander in Eingriff. Bevorzugt wird folglich durch die Magnete eine Kraft ausgeübt, die die beiden Formschlusselemente voneinander weg hält. Dies kann geschehen, indem die Magnete eine Kraft aufeinander ausüben. Dies kann beispielsweise eine abstoßende Kraft sein. Dies wird erreicht, indem ein Magnet des Beckenelementes und ein Magnet des Oberschenkelelementes nahe aneinander positioniert sind, sodass ihre gleichnamigen Pole, also jeweils Südpol oder jeweils Nordpol, aufeinander zu gerichtet sind. Wird nun das Beckenelement relativ zum Oberschenkelelement bewegt, werden auch die an den jeweiligen Elementen angeordneten Magnete bewegt. Es kommt folglich zu einer Verschiebung der bewegten Magnete zueinander. In dem Moment, in dem der Winkel des Oberkörperelementes relativ zum Beckenelement den vorbestimmten Grenzwinkel passiert, kommt vorzugsweise ein zweiter Magnet des Beckenelementes oder des Oberschenkelelementes in den Bereich des wenigstens einen Magnetes des jeweils anderen Elementes. Dabei kommt es nun zu einer anziehenden Kraft, da ungleichnamige Pole der beiden Magnete zueinander gerichtet werden.

Vorzugsweise verfügen zumindest einige, besonders bevorzugt jedoch alle, Vorsprünge und/oder Vertiefungen eines der Formschlusselemente, bevorzugt jedoch beider Formschlusselemente, über eine Hinterschnitt-Verzahnung. Dies bedeutet, dass vorzugsweise beide Flanken einer Vertiefung und/oder eines Vorsprunges in die gleiche Richtung geneigt sind. Dadurch kann allein durch die übertragenen Kräfte ein Drehmoment auf eines der Formschlusselemente ausgeübt werden, das in eine Kraft überführt wird, die eine axiale Komponente hat. Dadurch werden die beiden Formschlusselemente näher aufeinander zu gezogen, sodass die Stärke der Verzahnung, also des Eingriffs der beiden Formschlusselemente ineinander, erhöht wird.

Vorzugsweise sind die Formschlusselemente miteinander in Eingriff bringbar, in dem eines der Formschlusselemente auf das andere Formschlusselement zu bewegt wird, das relativ zu dem Bauteil, an dem es angeordnet ist, in einer Richtung drehbar gelagert ist. Dabei handelt es sich bevorzugt um eine schwimmende Lagerung, die eine geringfügige Rotation um beispielsweise weniger als 15°, bevorzugt weniger als 10°, besonders bevorzugt weniger als 5°, ermöglicht und so sicherstellt, dass die optimale Position und Orientierung der beiden Formschlusselemente zueinander erreicht werden kann.

Mithilfe der beigefügten Zeichnungen werden nachfolgend Ausführungsbeispiele der vorliegenden Erfindung näher erläutert. Es zeigen
- Figuren 1 und 3 -: eine Seitenansicht und eine Rückansicht eines Teils einer orthopädietechnischen Einrichtung gemäß einem ersten Ausführungsbeispiel der vorliegenden Erfindung,
- Figuren 2 und 4 -: vergrößerte Darstellungen von Teilen der Figuren 1 und 3,
- Figuren 5 bis 7 -: eine orthopädietechnische Einrichtung gemäß einem Ausführungsbeispiel der vorliegenden Erfindung im angelegten Zustand und,
- Figur 8 -: die schematische Darstellung unterschiedlicher Kraftverläufe als Funktion des Winkels zwischen Oberschenkelelement und Oberkörperelement.

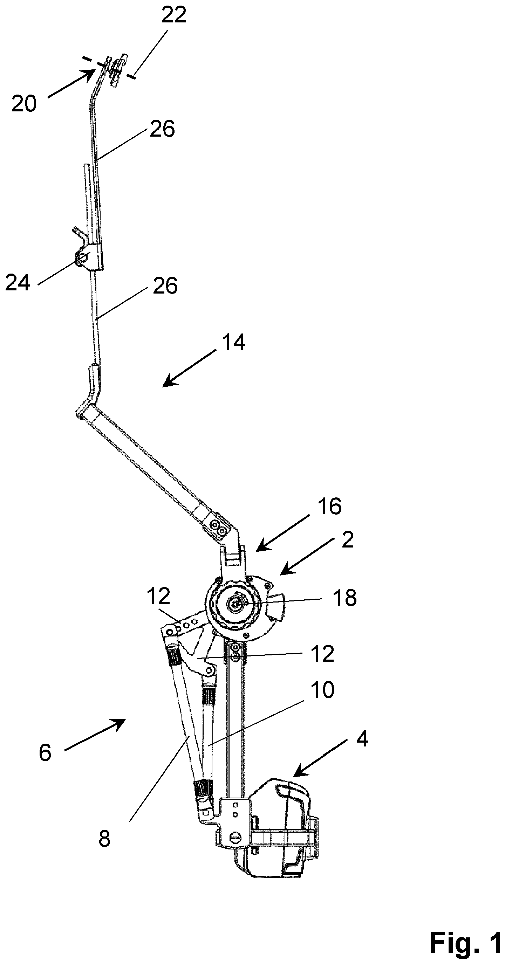

Figur 1 zeigt eine Seitenansicht eines Teils einer orthopädietechnischen Einrichtung gemäß einem ersten Ausführungsbeispiel der vorliegenden Erfindung. Die orthopädietechnische Einrichtung verfügt über ein Beckenelement 2, ein Oberschenkelelement 4 und einen mechanischen Energiespeicher 6. Der mechanische Energiespeicher 6 verfügt über einen ersten passiven Aktuator 8 und einen zweiten passiven Aktuator 10, die über zwei Krafteinleitungshebel 12 drehfest mit dem Beckenelement 2 verbindbar sind. Am Beckenelement 2 befindet sich ein Schienenelement 14, dessen erstes Ende 16 um eine erste Schwenkachse 18 schwenkbar am Beckenelement 2 angeordnet ist. Die erste Schwenkachse 18 erstreckt sich im gezeigten Ausführungsbeispiel senkrecht zur Zeichenebene. Ein zweites Ende 20 ist um eine zweite Schwenkachse 22 schwenkbar an einem nicht dargestellten Oberkörperelement angeordnet. Über eine Verstelleinrichtung 24, die im gezeigten Ausführungsbeispiel als Klemm Einrichtung ausgebildet ist, lässt sich die Länge des Schienenelementes 14 einstellen. Dazu werden im gezeigten Ausführungsbeispiel zwei Teilstücke 26 gegeneinander verschoben, sobald die Verstelleinrichtung 24 gelöst wurde. Anschließend wird die Verstelleinrichtung wieder arretiert und das Schienenelement 14 in dem geänderten Länge verwendet.

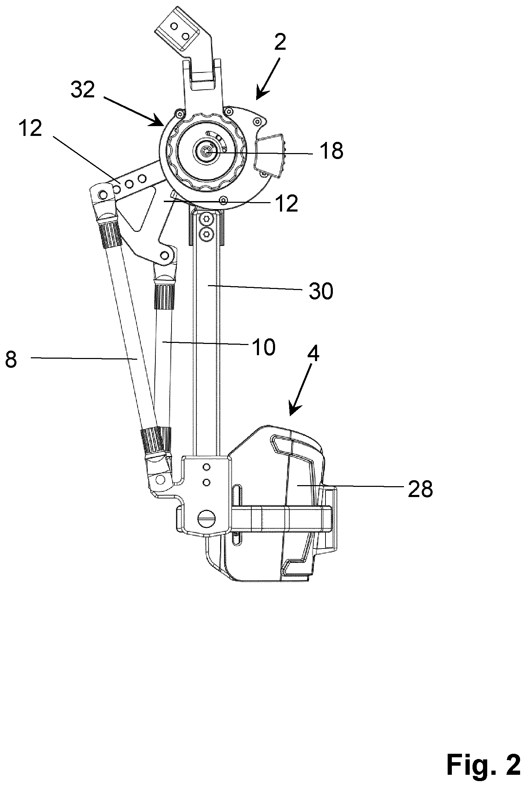

Figur 2 zeigt einen vergrößerten Ausschnitt aus Figur 1. Das Oberschenkelelement 4 verfügt über eine Oberschenkelschale 28, die an einem Abstandselement 30 angeordnet ist. Eine Gelenkanordnung 32 erlaubt es, das Oberschenkelelement 4 relativ zum Beckenelement 2 zu verschwenken. Die Gelenkanordnung 32 ist vorzugsweise in eine passive Stellung und in eine aktive Stellung bringbar. In der passiven Stellung sind die Krafteinleitungshebel 12 relativ zum Rest des Beckenelementes 2 bewegbar. Wird in diesem Zustand das Oberschenkelelement 4 relativ zum Beckenelement 2 verschwenkt, wird durch den ersten passiven Aktuator 8 und den zweiten passiven Aktuator 10 eine Kraft auf die Krafteinleitungshebel 12 aufgebracht, die dafür sorgt, dass die Krafteinleitungshebel 12 mit dem Oberschenkelelement 4 verschwenkt werden. Im aktiven Zustand der Gelenkanordnung 32 sind die Krafteinleitungshebel 12 drehfest mit dem Beckenelement 2 verbunden. Es ist daher nicht möglich, die Krafteinleitungshebel 12 mit dem Oberschenkelelement 4 zu verschwenken, wenn dieses relativ zum Beckenelement 2 bewegt wird. Daher werden die passiven Aktuatoren 8, 10 gespannt. Durch die unterschiedlichen Krafteinleitungshebel 12 und die unterschiedliche Länge der beiden passiven Aktuatoren 8, 10 enthalten die Aktuatoren bei unterschiedlichen Winkelstellungen unterschiedliche Kräfte.

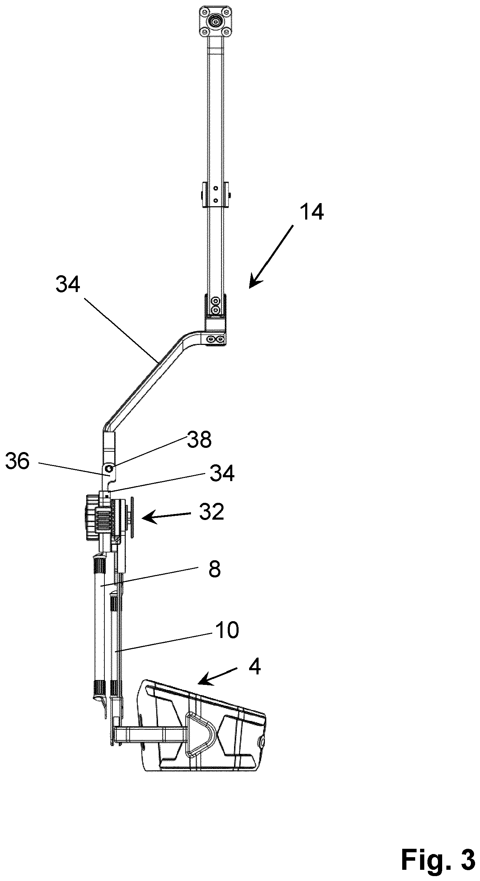

Figur 3 zeigt die orthopädietechnische Einrichtung aus Figur 1 in einer Rückansicht. Man erkennt das Oberschenkelelement 4 mit dem ersten passiven Aktuator 8 und dem zweiten passiven Aktuator 10, die Gelenkanordnung 32 sowie das Schienenelement 14. Das Schienenelement 14 verfügt über zwei Teilschienen 34 die durch ein Gelenk 36 mit einer dritten Schwenkachse 38 verbunden sind.

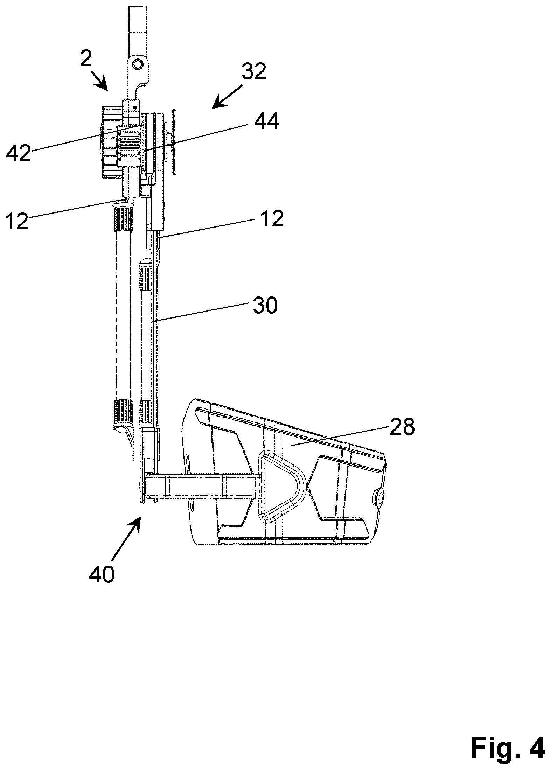

Figur 4 zeigt einen vergrößerten Ausschnitt aus Figur 3. Die Oberschenkelschale 28 ist durch eine Positioniereinrichtung 40 in wenigstens einer Richtung schwenkbar an dem Abstandselement 30 positioniert, sodass die optimale Position der Oberschenkelschale 28 relativ zum Oberschenkel des Benutzers ausgewählt werden kann. Die Gelenkeinrichtung 32 befindet sich im gezeigten Ausführungsbeispiel in der passiven Position. Man erkennt ein erstes Kraftübertragungselement 42 und ein zweites Kraftübertragungselement 44, die in der gezeigten Position nicht in Eingriff miteinander sind. Daher sind die Krafteinleitungshebel 12 nicht drehfest mit dem Beckenelement 2 verbunden.

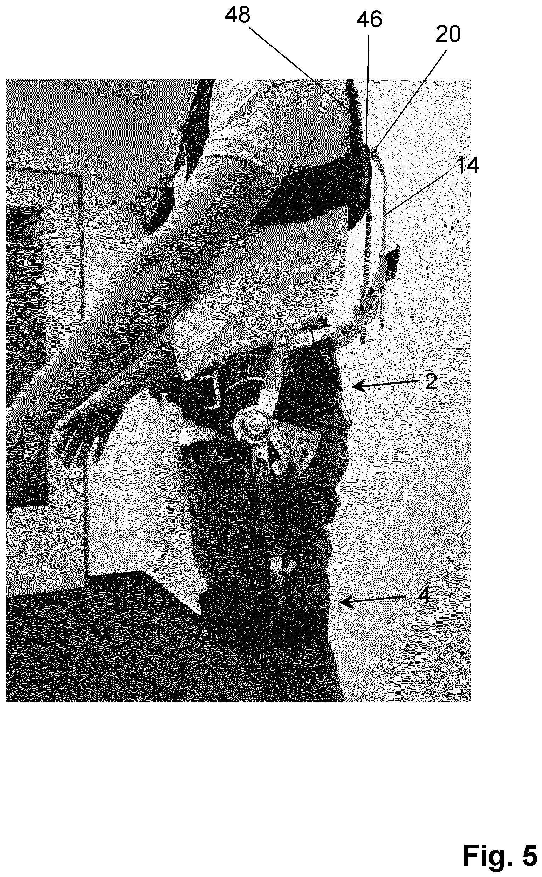

Die Figuren 5 bis 7 zeigen eine orthopädietechnische Einrichtung im angelegten Zustand. Figur 5 zeigt eine Seitenansicht des Benutzers. Man erkennt das Oberschenkelelement 4, dass Beckenelement 2 und insbesondere die Schienenelemente 14. Am zweiten Ende 20 sind die Schienenelemente über ein Kugelgelenk 46 am Oberkörperelement 48 angeordnet.

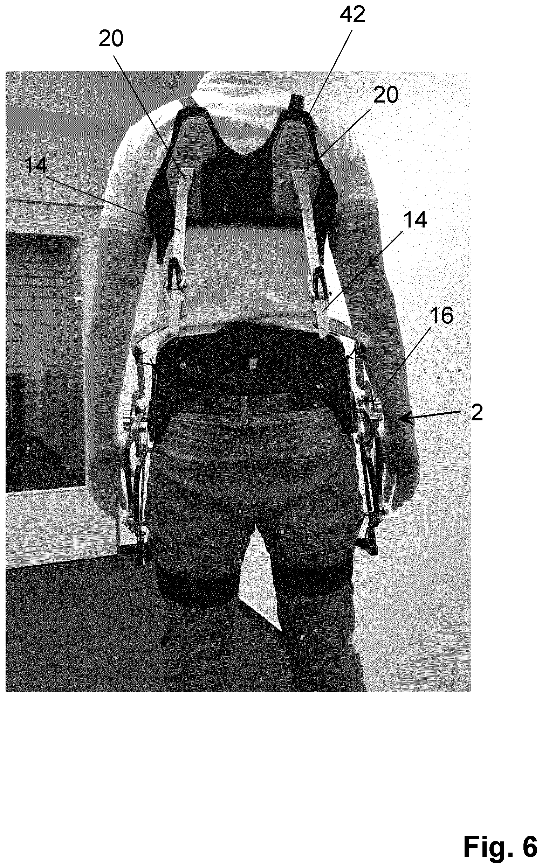

Figur 6 zeigt die orthopädietechnische Einrichtung im angelegten Zustand in einer Rückenansicht. Die beiden Schienenelemente 2 14 verlaufen von ihrem zweiten Ende 20 ausgehend vom Oberkörperelement 48 bis zum Beckenelement 2, an dem das erste Ende 16 angeordnet ist. Man erkennt, dass die zweiten Enden 20 dorsal, also am Rücken, am Oberkörperelement 48 angeordnet sind, während die ersten Enden 16 lateral, also seitlich, am Beckenelement 2 angeordnet sind.

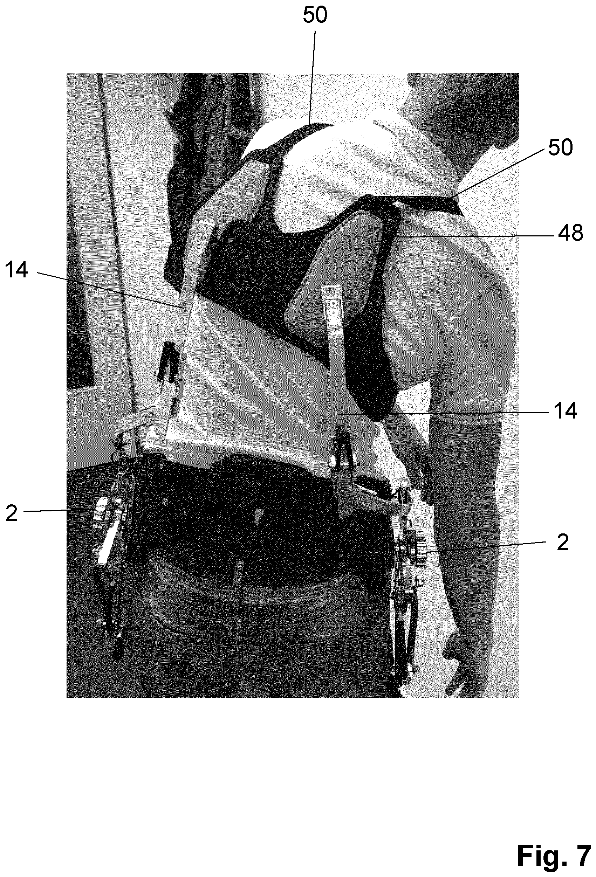

Figur 7 zeigt die Situation mit einer nach rechts geneigten Wirbelsäule. Das Oberkörperelement 48 verschiebt sich relativ zum Oberkörper leicht, was durch die beiden Gurte 50, die auch als Schultergurte bezeichnet werden können, ermöglicht wird. Gleichzeitig verschwenken die Schienenelemente 14 relativ zum Beckenelement 2 und erlauben so eine große Bewegungsfreiheit. Sowohl das Oberkörperelement 48 als auch das Beckenelement 2, zudem ein Beckengurt 52 gehört, sind in ihrer Länge einstellbar und somit für unterschiedliche Personen verwendbar.

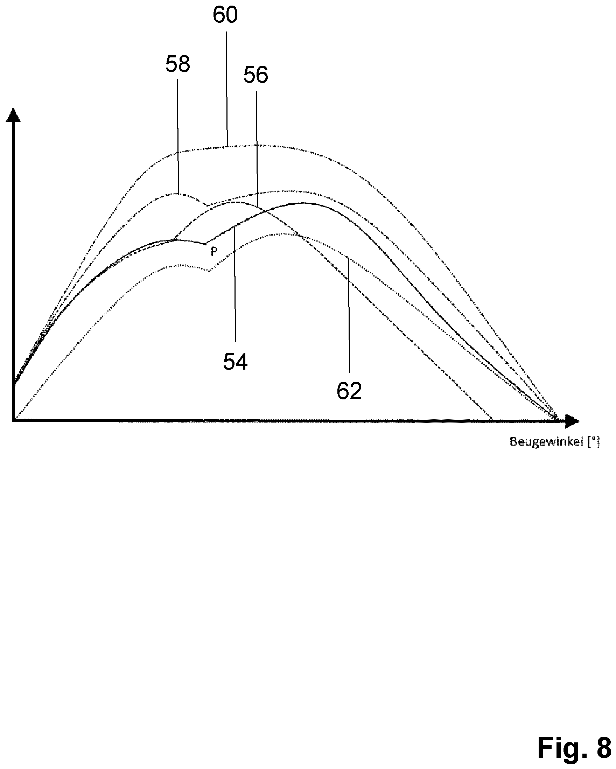

Figur 8 zeigt verschiedene Kraftverläufe, die auch als Drehmomentverläufe interpretiert werden können, der durch den ersten Aktuator 8 und den zweiten Aktuator 10 aufgebrachten Kraft als Funktion des Winkels zwischen dem Oberschenkelelement 4 und dem Oberkörperelement 48. Die durchgezogene Linie 54 beginnt nicht im Ursprung des Koordinatensystems. Die aufgebrachte Kraft ist folglich auch außerhalb des ersten vorbestimmten Winkelbereiches nicht gleich 0, sofern der Winkel größer ist als der vorbestimmte Winkelbereich. Der Winkel ist Beispielweise dann größer, wenn die Person aufrecht steht. Im gezeigten Ausführungsbeispiel ist der erste passiver Aktuator 8 folglich vorgespannt und übt eine Kraft aus. Diese Kraft steigt mit steigendem Beugewinkel, also kleinerem Winkel zwischen dem Oberschenkelelement 4 und dem Oberkörperelement 48, an, bis sie ein erstes Maximum erreicht. Bei weitere Beugung, also weitere Reduzierung des Winkels, sinkt die Kraft wieder ab, bis am Punkt P die Kraft wieder ansteigt. In diesem Bereich beginnt der vorbestimmte zweite Winkelbereich, indem der zweite passiver Aktuator 10 seine Kraft aufbringt. Auf diese steigt zunächst an, bis die durchgezogene Linie 54 ihr globales Maximum erreicht. Bei diesem Beugewinkel eben sowohl der erste passiver Aktuator 8 als auch der zweite passiver Aktuator 10 eine Kraft aus. Diese reduzieren sich bei weiterer Beugung soweit, bis am Ende keine Kraft mehr ausgeübt wird.

Die gestrichelte Kurve 56 zeigt eine ähnliche Situation. Im Unterschied zur durchgezogenen Linie 54 ist der Winkel zwischen den beiden Krafteinleitungshebel 12 verkleinert worden. Dies wird beispielsweise erreicht, in dem der Krafteinleitungshebel 12, an dem der zweite passiver Aktuator 10 angreift, in den Figuren 1 und 2 gegen den Uhrzeigersinn relativ zum zweiten Krafteinleitungshebel 12, an dem der erste passiver Aktuator 8 angreift, verschoben wird. Man erkennt, dass der Punkt P nach links verschoben ist. Der zweite passiver Aktuator 10 bildet folglich bereits bei kleineren Beugewinkel, also größeren Winkeln zwischen dem Oberschenkelelement 4 und dem Oberkörperelement 48, seine Kraft aus.

Die Strich-Punkt-Linie 58 zeigt eine weitere Situation. Im Vergleich zur durchgezogenen Linie 54 wurde die Position der beiden Krafteinleitungshebel 12 relativ zueinander nicht verändert. Vielmehr wurden die beiden Krafteinleitungshebel 12 verlängert, wodurch die vom ersten passiven Aktuator 8 und vom zweiten passiven Aktuator 10 aufgebrachte Kraft vergrößert wird, weil der Hebelarm sich verlängert.

Die Strich-Punkt-Punkt-Linie 60 zeigt die Situation, in der sowohl der erste passiver Aktuator 8 als auch der zweite passiver Aktuator 10 über den gesamten Bereich Ihre Kraftaufbringung.

Die gepunktete Linie 62 entspricht der parallel verschobenen durchgezogene Linie 54. Der erste passiver Aktuator 8 wird folglich ohne Vorspannung verwendet.

### Bezugszeichenliste

- 2: Beckenelement
- 4: Oberschenkelelement
- 6: mechanischer Energiespeicher
- 8: erster passiver Aktuator
- 10: zweiter passiver Aktuator
- 12: Krafteinleitungshebel
- 14: Schienenelement
- 16: erstes Ende
- 18: erste Schwenkachse
- 20: zweites Ende
- 22: zweite Schwenkachse
- 24: Verstelleinrichtung
- 26: Teilstück
- 28: Oberschenkelschale
- 30: Abstandselement
- 32: Gelenkanordnung
- 34: Teilschiene
- 36: Gelenk
- 38: dritte Schwenkachse
- 40: Positioniereinrichtung
- 42: erstes Kraftübertragungselement
- 44: zweites Kraftübertragungselement
- 46: Kugelgelenk
- 48: Oberkörperelement
- 50: Gurt
- 52: Beckengurt
- 54: durchgezogene Linie
- 56: gestrichelte Kurve
- 58: Strich-Punkt-Linie
- 60: Strich-Punkt-Punkt-Linie
- 62: gepunktete Linie

## Patentansprüche

1. Orthopädietechnische Einrichtung zum Unterstützen eines unteren Rückens eines Benutzers, wobei die orthopädietechnische Einrichtung
- wenigstens einen mechanischen Energiespeicher (6),
- ein Beckenelement (2),
- ein Oberkörperelement (48) und
- ein Oberschenkelelement (4) aufweist,
wobei der mechanische Energiespeicher (6) aufladbar und entladbar ist, indem das Oberschenkelelement (4) relativ zu dem Oberkörperelement (48) verschwenkt wird,
wobei das Oberkörperelement (48) mittels zweier Schienenelemente (14) an dem Beckenelement (2) angeordnet ist, wobei die Schienenelemente (14) jeweils mit einem ersten Ende (16) um wenigstens eine erste Schwenkachse (18) schwenkbar an dem Beckenelement (2) und mit einem dem ersten Ende (16) entgegengesetzten zweiten Ende (20) um wenigstens eine zweite Schwenkachse (22) schwenkbar an dem Oberkörperelement (48) angeordnet sind,
wobei die ersten Schwenkachsen (18) durch die Hüftgelenke des Benutzers verlaufen, so dass die ersten Enden (16) der Schienenelemente (14) lateral angeordnet sind, **dadurch gekennzeichnet,**
**dass** die zweiten Enden (20) der
Schienenelemente (14) im angelegten Zustand der Vorrichtung im Bereich der Schulterblätter des Benutzers angeordnet sind .

2. Orthopädietechnische Einrichtung nach Anspruch 1 , **dadurch gekennzeichnet, dass** die zweiten Schwenkachsen (22) zumindest im Wesentlichen in Sagittalebenen verlaufen.

3. Orthopädietechnische Einrichtung nach einem der vorstehenden Ansprüche, **dadurch gekennzeichnet, dass** die Schienenelemente (14) jeweils wenigstens zwei Teilschienen (34) aufweisen, die um eine dritte Schwenkachse (38) schwenkbar aneinander angeordnet sind, wobei die dritten Schwenkachsen (38) zumindest im Wesentlichen in Sagittalebenen verlaufen.

4. Orthopädietechnische Einrichtung nach einem der vorstehenden Ansprüche, **dadurch gekennzeichnet, dass** die zweiten Enden (20) der Schienenelemente (14) im angelegten Zustand der Vorrichtung im Bereich der unteren Winkel der Schulterblätter des Benutzers angeordnet sind.

5. Orthopädietechnische Einrichtung nach einem der vorstehenden Ansprüche, **dadurch gekennzeichnet, dass** ein Abstand zwischen Gelenken, mit denen die zweiten Enden (20) der Schienenelemente (14) am Oberkörperelement (48) angeordnet sind, einstellbar ist.

6. Orthopädietechnische Einrichtung nach einem der vorstehenden Ansprüche, **dadurch gekennzeichnet, dass** die zweiten Enden (20) mittels Kugelgelenken (46) an dem Oberkörperelement (48) angeordnet sind.

7. Orthopädietechnische Einrichtung nach einem der vorstehenden Ansprüche, **dadurch gekennzeichnet, dass** das Oberkörperelement (48) im angelegten Zustand der orthopädietechnische Einrichtung einen Oberkörper des Benutzers umgibt und derart formstabil ausgebildet ist, dass sich ein Durchmesser des Oberkörperelementes (48) in medial-lateral-Richtung und/oder in dorsal-ventral-Richtung beim Beugen des Oberkörpers nicht oder im Wesentlichen nicht verringert.

8. Orthopädietechnische Einrichtung nach Anspruch 6, **dadurch gekennzeichnet, dass** das Oberkörperelement (48) einen Brustabschnitt aufweist, der im angelegten Zustand der orthopädietechnische Einrichtung an wenigstens zwei voneinander beabstandeten Stellen auf unterschiedlichen Seiten eines Brustbeines des Benutzers an der Brust des Benutzers anliegt.

9. Orthopädietechnische Einrichtung nach einem der vorstehenden Ansprüche, **dadurch gekennzeichnet, dass** die orthopädietechnische Einrichtung ein erstes und ein zweites Oberschenkelelement (4) und wenigstens einen ersten und wenigstens einen zweiten mechanischen Energiespeicher (6) aufweist, wobei der erste mechanische Energiespeicher (6) aufladbar und entladbar ist, indem das erste Oberschenkelelement (4) relativ zu dem Oberkörperelement (48) verschwenkt wird und wobei der zweite mechanische Energiespeicher (6) aufladbar und entladbar ist, indem das zweite Oberschenkelelement (4) relativ zu dem Oberkörperelement (48) verschwenkt wird.

10. Orthopädietechnische Einrichtung nach einem der vorstehenden Ansprüche, **dadurch gekennzeichnet, dass** jedes Oberschenkelelement (4) mittels einer Gelenkanordnung (32) um eine Gelenkachse schwenkbar an dem Beckenelement (2) angeordnet ist.

11. Orthopädietechnische Einrichtung nach einem der vorstehenden Ansprüche, **dadurch gekennzeichnet, dass** jedes Schienenelement (14) um wenigstens zwei Schwenkachsen (18, 22, 38) schwenkbar an dem Oberkörperelement (48) angeordnet ist, wobei wenigstens zwei der Schwenkachsen (18, 22, 38) vorzugsweise senkrecht aufeinander stehen.

12. Orthopädietechnische Einrichtung nach einem der vorstehenden Ansprüche, **dadurch gekennzeichnet, dass** wenigstens ein Schienenelement (14) mittels eines Kugelgelenkes (46) an dem Oberkörperelement (48) angeordnet ist.

13. Orthopädietechnische Einrichtung nach einem der vorstehenden Ansprüche, **dadurch gekennzeichnet, dass** wenigstens ein Schienenelement (14) längenveränderlich ausgebildet ist.

14. Orthopädietechnische Einrichtung nach einem der vorstehenden Ansprüche, **dadurch gekennzeichnet, dass** an dem Oberschenkelelement (4) eine Oberschenkelschale (28) zum Anlegen an den Oberschenkel mittels eines Kugelgelenkes (46) angeordnet ist.

15. Orthopädietechnische Einrichtung nach Anspruch 14, **dadurch gekennzeichnet, dass** die Oberschenkelschale (28) relativ zu dem Oberschenkelelement (4) um eine Rotationsachse schwenkbar, bevorzugt gegen eine Kraft eines Federelementes schwenkbar, ist, wobei sich die vorzugsweise Rotationsachse in medial-lateral-Richtung erstreckt.

## Claims

1. An orthopedic device for supporting a lower back of a user, wherein the orthopedic device comprises
- at least one mechanical energy store (6),
- a pelvic element (2),
- an upper body element (48) and
- an upper leg element (4),
wherein the mechanical energy store (6) can be charged and discharged by swiveling the upper leg element (4) relative to the upper body element (48), wherein the upper body element (48) is arranged on the pelvic element (2) by means of two splint elements (14), wherein the splint elements (14) are each arranged with a first end (16) on the pelvic element (2) such that they can be swiveled about at least a first swivel axis (18) and with a second end (20) opposite the first end (16) on the upper body element (48) such that they can be swiveled about at least a second swivel axis (22),
wherein the first swivel axes (18) extend through the hip joints of the user, so that the first ends (16) of the splint elements (14) are arranged laterally, **characterized in that**, when the orthopedic device is mounted, the second ends (20) of the splint elements (14) are arranged in the region of the shoulder blades of the user.

2. The orthopedic device according to claim 1, **characterized in that** the second swivel axes (22) extend at least largely in sagittal planes.

3. The orthopedic device according to one of the preceding claims, **characterized in that** the splint elements (14) each comprise at least two partial splints (34), which are arranged on each other such that they can be swiveled about a third swivel axis (38), wherein the third swivel axes (38) extend at least largely in sagittal planes.

4. The orthopedic device according to one of the preceding claims, **characterized in that**, when the orthopedic device is mounted, the second ends (20) of the rail elements (14) are arranged in the region of the lower angles of the user's shoulder blades.

5. The orthopedic device according to one of the preceding claims, **characterized in that** a distance between joints, with which the two ends (20) of the splint elements (14) are arranged on the upper body element (48), is adjustable.

6. The orthopedic device according to one of the preceding claims, **characterized in that** the second ends (20) are arranged on the upper body element (48) by means of ball joints (46).

7. The orthopedic device according to one of the preceding claims, **characterized in that**, when the orthopedic device is mounted, the upper body element (48) surrounds an upper body of the user and is designed to be so dimensionally stable that a diameter of the upper body element (48) in the medial-lateral direction and/or dorsal-ventral direction does not or largely does not decrease when the upper body bends over.

8. The orthopedic device according to claim 6, **characterized in that** the upper body element (48) comprises a chest section which, when the orthopedic device is mounted, rests on the user's chest at at least two spaced points on different sides of the user's sternum.

9. The orthopedic device according to one of the preceding claims, **characterized in that** the orthopedic device comprises a first and a second upper leg element (4), and at least a first and a second mechanical energy store (6), wherein the first mechanical energy store (6) can be charged and discharged by swiveling the first upper leg element (4) relative to the upper body element (48), and wherein the second mechanical energy store (6) can be charged and discharged by swiveling the second upper leg element (4) relative to the upper body element (48).

10. The orthopedic device according to one of the preceding claims, **characterized in that** each upper leg element (4) is arranged on the pelvic element (2) by means of a joint arrangement (32) such that it can be swiveled about a joint axis.

11. The orthopedic device according to one of the preceding claims, **characterized in that** each splint element (14) is arranged on the upper body element (48) such that it can be swiveled about at least two swivel axes (18, 22, 38), wherein at least two of the swivel axes (18, 22, 38) are preferably perpendicular to one another.

12. The orthopedic device according to one of the preceding claims, **characterized in that** at least one splint element (14) is arranged on the upper body element (48) by means of a ball joint (46).

13. The orthopedic device according to one of the preceding claims, **characterized in that** at least one splint element (14) is designed to be adjustable in length.

14. The orthopedic device according to one of the preceding claims, **characterized in that** an upper leg shell (28) for mounting on the upper leg is arranged on the upper leg element (4) by means of a ball joint (46).

15. The orthopedic device according to claim 14, **characterized in that** the upper leg shell (28) can be swiveled relative to the upper leg element (4) about a rotational axis, preferably against a force of a spring element, wherein the rotational axis preferably extends in the medial-lateral direction.

## Revendications

1. Dispositif orthopédique pour soutenir le bas du dos d'un utilisateur, le dispositif orthopédique comprenant
- au moins un accumulateur d'énergie mécanique (6),
- un élément de bassin (2),
- un élément de torse (48), et
- un élément de cuisse (4),
dans lequel
l'accumulateur d'énergie mécanique (6) peut être chargé et déchargé par pivotement de l'élément de cuisse (4) par rapport à l'élément de torse (48), l'élément de torse (48) est agencé sur l'élément de bassin (2) au moyen de deux éléments de rail (14), les éléments de rail (14) étant disposés chacun avec une première extrémité (16) sur l'élément de bassin (2) de manière à pouvoir pivoter autour d'au moins un premier axe de pivotement (18) et avec une deuxième extrémité (20), opposée à la première extrémité (16), sur l'élément de torse (48) de manière à pouvoir pivoter autour d'au moins un deuxième axe de pivotement (22),
les premiers axes de pivotement (18) passent par les articulations de la hanche de l'utilisateur, de sorte que les premières extrémités (16) des éléments de rail (14) sont agencées latéralement,
**caractérisé en ce que** les deuxièmes extrémités (20) des éléments de rail (14) sont agencées, à l'état appliqué du dispositif, dans la zone des omoplates de l'utilisateur.

2. Dispositif orthopédique selon la revendication 1,
**caractérisé en ce que** les deuxièmes axes de pivotement (22) s'étendent au moins sensiblement dans des plans sagittaux.

3. Dispositif orthopédique selon l'une des revendications précédentes, **caractérisé en ce que** les éléments de rail (14) présentent chacun au moins deux rails partiels (34) qui sont agencés l'un contre l'autre de manière à pouvoir pivoter autour d'un troisième axe de pivotement (38), les troisièmes axes de pivotement (38) s'étendant au moins sensiblement dans des plans sagittaux.

4. Dispositif orthopédique selon l'une des revendications précédentes, **caractérisé en ce que** les deuxièmes extrémités (20) des éléments de rail (14) sont agencées, à l'état appliqué du dispositif, dans la zone des angles inférieurs des omoplates de l'utilisateur.

5. Dispositif orthopédique selon l'une des revendications précédentes, **caractérisé en ce qu'**une distance entre les articulations, par lesquelles les deuxièmes extrémités (20) des éléments de rail (14) sont agencées sur l'élément de torse (48), est réglable.

6. Dispositif orthopédique selon l'une des revendications précédentes, **caractérisé en ce que** les deuxièmes extrémités (20) sont agencées sur l'élément de torse (48) au moyen d'articulations à rotule (46).

7. Dispositif orthopédique selon l'une des revendications précédentes, **caractérisé en ce qu'**à l'état appliqué du dispositif orthopédique, l'élément de torse (48) entoure le torse de l'utilisateur et est conçu avec une stabilité de forme telle qu'un diamètre de l'élément de torse (48) dans la direction médiale-latérale et/ou dans la direction dorsale-ventrale ne diminue pas ou sensiblement pas lors d'une flexion du torse.

8. Dispositif orthopédique selon la revendication 6,
**caractérisé en ce que** l'élément de torse (48) présente une partie de poitrine qui, à l'état appliqué du dispositif orthopédique, repose sur la poitrine de l'utilisateur en au moins deux endroits espacés l'un de l'autre sur différents côtés d'un sternum de l'utilisateur.

9. Dispositif orthopédique selon l'une des revendications précédentes, **caractérisé en ce que** le dispositif orthopédique comprend un premier et un deuxième élément de cuisse (4) et au moins un premier et au moins un deuxième accumulateur d'énergie mécanique (6), le premier accumulateur d'énergie mécanique (6) pouvant être chargé et déchargé par pivotement du premier élément de cuisse (4) par rapport à l'élément de torse (48), et le deuxième accumulateur d'énergie mécanique (6) pouvant être chargé et déchargé par pivotement du deuxième élément de cuisse (4) par rapport à l'élément de torse (48).

10. Dispositif orthopédique selon l'une des revendications précédentes, **caractérisé en ce que** chaque élément de cuisse (4) est agencé sur l'élément de bassin (2) de manière à pouvoir pivoter autour d'un axe d'articulation au moyen d'un ensemble d'articulation (32).

11. Dispositif orthopédique selon l'une des revendications précédentes, **caractérisé en ce que** chaque élément de rail (14) est agencé sur l'élément de torse (48) de manière à pouvoir pivoter autour d'au moins deux axes de pivotement (18, 22, 38), au moins deux des axes de pivotement (18, 22, 38) étant de préférence perpendiculaires l'un à l'autre.

12. Dispositif orthopédique selon l'une des revendications précédentes, **caractérisé en ce qu'**au moins un élément de rail (14) est agencé sur l'élément de torse (48) au moyen d'une articulation à rotule (46).

13. Dispositif orthopédique selon l'une des revendications précédentes, **caractérisé en ce qu'**au moins un élément de rail (14) est réalisé de manière variable en longueur.

14. Dispositif orthopédique selon l'une des revendications précédentes, **caractérisé en ce qu'**une coque de cuisse (28), destinée à être appliquée sur la cuisse, est agencée sur l'élément de cuisse (4) au moyen d'une articulation à rotule (46).

15. Dispositif orthopédique selon la revendication 14,
**caractérisé en ce que** la coque de cuisse (28) peut pivoter par rapport à l'élément de cuisse (4) autour d'un axe de rotation, de préférence à l'encontre d'une force d'un élément à ressort, l'axe de rotation s'étendant de préférence dans la direction médiale-latérale.
